# EUROPÄISCHE PATENTSCHRIFT

(11) **EP 1 001 724 B1**
(45) Veröffentlichungstag und Bekanntmachung des Hinweises auf die Patenterteilung: **03.12.2003**
(21) Anmeldenummer: 98948725.1
(22) Anmeldetag: 04.08.1998
(51) Int. Cl.: A61F 5/453

(54) **VORRICHTUNG ZUR KOMBINIERTEN URINABLEITUNG UND KATHETERISIERUNG INKONTINENTER MÄNNLICHER PERSONEN**
DEVICE FOR COMBINED URINARY DRAINAGE AND CATHETERIZATION OF INCONTINENT MALE HUMAN BEINGS
DISPOSITIF POUR LA DERIVATION URINAIRE ET LE CATHETERISME COMBINES DE SUJETS MALES INCONTINENTS

(30) Priorität: 04.08.1997 DE 19733665
(43) Veröffentlichungstag der Anmeldung: 24.05.2000
(73) Patentinhaber: Sauer, Manfred, 74931 Lobbach (DE)
(72) Erfinder: Sauer, Manfred, 74931 Lobbach (DE)
(74) Vertreter: Naumann, Ulrich, Dr.-Ing.
(86) Internationale Anmeldenummer: DE9802241
(87) Internationale Veröffentlichungsnummer: WO99007313

(56) Entgegenhaltungen:
- EP-A- 0 068 712
- US-A- 2 601 547
- US-A- 4 370 979
- US-A- 4 484 918
- US-A- 5 275 587
- US-A- 5 312 383

## Beschreibung

Die Erfindung betrifft eine Vorrichtung zur kombinierten Urinableitung und Katheterisierung inkontinenter männlicher Personen, insbesondere bei Querschnittslähmung, mit einem im vorderen Bereich eine Öffnung aufweisenden Kondom zum Festkleben am Penisschaft, einer abdichtend an die Öffnung des Kondoms anschließbaren Anschlußeinrichtung zum lösbaren Verbinden mit einem Urinsammelbehältnis und einem Katheter, wobei zum Katheterisieren die Anschlußeinrichtung zu entfernen ist.

Eine Vorrichtung gemäß dem Oberbegriff von Anspruch 1 ist aus der DE-A-37 41 557 bekannt.

Zur Bewältigung der Inkontinenz männlicher Personen, insbesondere bei Querschnittslähmung, sind aus der Praxis im wesentlichen zwei unterschiedliche Methoden bekannt. So gibt es bereits ein urinableitendes System, bei dem ein kondomähnliches Tauch- oder Spritzgußteil aus einem elastischen Material, beispielsweise aus Silikon oder dergleichen, mit einem Kleber am Penisschaft rückflußsicher fixiert wird. Über eine Öffnung im vorderen Bereich des Kondoms und über eine dort vorgesehene Anschlußeinrichtung wird das Urin über einen Schlauch in einen Bett- oder Beinbeutel abgeleitet.

Alternativ zu dem voranstehend kurz erörterten urinableitenden System gibt es den sog. intermetierenden Selbstkatheterismus (ISK), wonach sich die inkontinente männliche Person vier bis sechs Mal am Tag selbst katheterisiert. Um zwischen den Katheterisierungsphasen kontinent zu bleiben wird üblicherweise vom behandelnden Arzt ein Medikament verordnet, welches die Blase reaktiviert bzw. ruhig stellt. Sämtliche hier in Frage kommenden Medikamente haben jedoch den ganz erheblichen Nachteil, daß sie die Schleimhäute des menschlichen Körpers austrocknen, was vom Patienten als unangenehme Wirkung oder gar schmerzverursachend empfunden wird.

Im Rahmen des intermetierenden Selbstkatheterismus könnte man unter Ausschluß der durch Medikamente hervorgerufenen Nebenwirkungen auf entsprechende Medikamente vollkommen verzichten, müßte jedoch zumindest zwischen den Katheterisierphasen ein urinableitendes System anschließen. Dies bedeutet, daß man mit einem hautverträglichen Kleber oder Klebestreifen ein Kondom wie bei dem herkömmlichen urinableitenden System am Penisschaft fixieren müßte. Je nach Häufigkeit des Katheterisierens - vier bis sechs Mal am Tag - müßte entsprechend oft das urinableitende System angeklebt und auch wieder entfernt werden. Auch bei hautverträglichen Klebern führt dies zu einer enormen Hautbelastung und schließlich durch den erhöhten Materialverbrauch zu ganz erheblichen Mehrkosten.

Der vorliegenden Erfindung liegt daher die Aufgabe zugrunde, eine Vorrichtung zur kombinierten Urinableitung und Katheterisierung inkontinenter männlicher Personen anzugeben, wonach das Kondom nur einmal am Tag, vorzugsweise früh morgens nach der Hygiene, mittels Klebstoff zu fixieren ist und wobei dennoch eine einfache und dabei hygienische Handhabung der Katheterisierung gewährleistet ist.

Die zuvor genannte Aufgabe ist durch eine Vorrichtung mit den Merkmalen des Patentanspruches 1 gelöst. Danach ist eine Vorrichtung zur kombinierten Urinableitung und Katheterisierung inkontinenter männlicher Personen der voranstehend erörterten Art gekennzeichnet durch eine zum Dehnen der Öffnung des Kondoms dienende Spreizeinrichtung, mit deren Hilfe die Öffnung so weit dehnbar ist, daß das Kondom von der Öffnung her gemeinsam mit der Spreizeinrichtung über die Eichel und zumindest einen Teil des Penisschafts hinweg dem Körper entgegen schiebbar ist.

Erfindungsgemäß ist erkannt worden, daß man auch bei mehrfachem täglichen Katheterisieren grundsätzlich ein urinableitendes System mit Kondom - unter Verzicht sonst üblicher Medikamente zur Deaktivierung der Blase - anlegen kann, wobei das Anlegen einmal am Tag, beispielsweise morgens nach der Hygiene, erfolgt. Zum Katheterisieren wird die am vorderen Ende ausgebildete, von Anschlußmitteln befreite Öffnung des Kondoms mittels einer besonderen Spreizeinrichtung geweitet und über den Penisschaft zurückgestreift, so daß die Eichel vollkommen frei liegt und vor der Katheterisierung gesäubert werden kann. Anschließend - nach dem Katheterisieren - wird das Kondom mittels der Spreizeinrichtung wieder über die Eichel nach vorne hinweggeschoben. Die Spreizeinrichtung wird wieder zusammengefahren, so daß sich die Öffnung entdehnen kann. Danach ist ein erneutes Festlegen der Anschlußeinrichtung möglich, wodurch der Anschluß des urinableitenden Systems zu bewerkstelligen ist.

Ein weiterer Vorteil der erfindungsgemäßen Vorrichtung ist darin zu sehen, daß die Spreizeinrichtung einerseits zur Handhabung des Kondoms und andererseits als Stütze für den Penisschaft während des Katheterisierens dient. Jedenfalls kann auf die beim intermetierenden Selbstkatheterismus üblicherweise einzunehmenden Medikamente verzichtet werden, wodurch die dort auftretenden Nebenwirkungen ausgeschlossen sind. Eine Katheterisierung in den gewünschten Intervallen ist ohne Medikamenteinnahme möglich, wobei es sich hier eben um eine Kombination von intermittierendem Selbstkatheterismus und Urinableitung handelt.

An dieser Stelle sei noch einmal angemerkt, daß die Spreizeinrichtung über ihre eigentliche Aufgabe hinaus auch zur Positionierung des Penisschafts während des Einführens des Katheters dient. Entsprechend könnte die Spreizeinrichtung im Sinne eines stützenden Teils ausgestaltet sein, wobei die Handhabbarkeit für Personen mit intakter Fingerfunktion, aber auch für Personen mit eingeschränkter Fingerfunktion gewährleistet sein muß.

Hinsichtlich einer konkreten Ausgestaltung der Spreizeinrichtung ist es von Vorteil, wenn diese als Spreizring ausgeführt ist. Wie bereits zuvor ausgeführt, wird zur Katheterisierung die kleine Öffnung des Kondoms von der Anschlußeinrichtung befreit, wird danach - bei Anwendung der Spreizeinrichtung - von der Spreizeinrichtung erfaßt und soweit geöffnet, daß das Kondom gemeinsam mit dem Spreizring über die Eichel und den Penisschaft hinaus dem Körper entgegen zurückgestülpt werden kann, um nämlich dadurch den gesamten Eichelbereich freizulegen, und zwar einerseits zur Reinigen bzw. zur Desinfektion und andererseits zum anschließenden Einführen des Katheters. Während der Katheterisierung gibt die als Spreizring ausgebildete Spreizeinrichtung dem Penisschaft einen hinreichenden Halt, so daß die Katheterisierung auch bei eingeschränkter Fingerbeweglichkeit durch die so erzielte Hilfestellung möglich ist.

Für die Spreizeinrichtung bzw. für den Spreizring ist des weiteren wesentlich, daß es sich hier um ein mehrfach bzw. dauerhaft verwendbares Hilfsmittel handelt. Gleiches gilt für die später noch zu erörternde Anschlußeinrichtung.

Hinsichtlich der konkreten Ausgestaltung der Spreizeinrichtung - ausgebildet im Sinne eines Spreizrings - weist die Spreizeinrichtung in etwa koaxial kreisringförmig angeordnete Spreizfinger zum Einführen in die ungedehnte Öffnung des Kondoms auf, wobei die Spreizfinger zum Dehnen der Öffnung auf einen größeren Radius auseinander bewegbar sind. Vor der Anwendung der Spreizeinrichtung ist selbstverständlich die Anschlußeinrichtung zu lösen und vom Kondom zu entfernen.

Genauer gesagt könnten die Spreizfinger derart angeordnet sein, daß sie blendenartig auseinander und aufeinander zu bewegbar sind. Insoweit ließe sich die Öffnung des Kondoms quasi stufenlos dehnen und auch wieder durch blendenartiges Zusammenfahren verkleinern, so daß anschließend wieder die Anschlußeinrichtung an der Öffnung anbringbar ist.

Die zum Eingreifen in die Öffnung des Kondoms dienenden Spreizfinger ragen in etwa orthogonal von der Spreizeinrichtung ab und sind über eine Hebelanordnung an die Spreizeinrichtung angelenkt. Jeder Spreizfinger könnte dabei fest mit einem schwenkbaren Hebelarm verbunden sein, wobei Spreizfinger und Hebelarm in etwa L-förmig verlaufen.

Die Spreizeinrichtung bzw. der Spreizring könnte in weiter vorteilhafter Weise zwei gegeneinander drehbare Ringscheiben umfassen, wobei die Ringscheiben gegenseitig in Führungsorgane eingreifen und dabei drehbar miteinander verbunden sind. Die Drehverbindung könnte über herkömmliche Rastelemente, Rastnasen, Sprengringe oder dergleichen bewerkstelligt sein, wobei die Hebelanordnung und somit auch die Hebelarme zwischen den Ringscheiben in ihrer Schwenkbewegung zwangsgeführt sind.

Im konkreten könnten die Hebelarme an einer der Ringscheiben schwenkbar angelenkt und an der jeweils anderen Ringscheibe durch Führungselemente derart geführt sein, daß durch gegenseitiges Verdrehen der Ringscheiben die Hebelarme um einen vorgebbaren Bereich geschwenkt werden. Dabei sind die Spreizfinger von einer kreisringförmigen Anordnung mit kleinem Durchmesser in eine kreisringförmige Anordnung mit größerem Durchmesser und umgekehrt verlagerbar, wodurch sich die zum Dehnen und auch wieder Entdehnen erforderliche Bewegung der Spreizfinger im Sinne der Bewegung einer Blende ergibt.

Die Führung der Hebelarme zwischen den beiden Ringscheiben läßt sich auf verschiedene Arten realisieren: So könnten die Führungselemente als die Hebelarme führende Laschen ausgeführt sein, wobei diese Laschen derart konstruiert und angeordnet sein müßten, daß sie eine Schwenk- und Kippbewegung der Führungselemente zulassen. Für entsprechendes Spiel ist demnach zu sorgen.

Ebenso könnten die Führungselemente - in ganz besonders vorteilhafter Weise - als in Führungsschlitze der Hebelarme eingreifende Stifte ausgeführt sein, wobei dadurch - per se - eine Schwenkbewegung der Hebelarme definiert ist. Da die Führungselemente in längs der Hebelarme ausgebildete Führungsschlitze eingreifen, ist eine Verlagerung der Schwenkachse - definierte durch die Führungselemente - möglich.

Hinsichtlich einer einfachen Betätigung der Spreizeinrichtung ist es von Vorteil, wenn die Ringscheiben am Außenrand Griffbereiche zur besseren Handhabung aufweisen. Dabei könnte es sich um Profilierungen der Außenwandung der Ringscheiben handeln. Insbesondere dann, wenn die Spreizeinrichtung von Personen mit eingeschränkter Fingerfunktion betätigbar sein soll, könnten besondere Betätigungsmechanismen vorgesehen sein. Insoweit könnten die Ringscheiben über einen um diese herum angeordneten Dreh-/Druckmechanismus auf Druck gegeneinander verdrehbar sein, wonach nämlich eine lineare Bewegung in eine Drehbewegung der Ringscheiben umgesetzt wird. Die die Spreizeinrichtung anwendende Person müßte somit lediglich die Spreizfinger in die Öffnung des Kondoms einführen und den Dreh-/Druckmechanismus von außen zusammendrücken, wodurch die Ringscheiben gegeneinander verdreht und die Spreizfinger in eine ringförmige Anordnung mit größerem Radius verbracht werden. Eine Arretierung der Spreizfinger in dieser Position sollte möglich sein.

Insbesondere im Hinblick auf eine einfache konstruktive Ausgestaltung ist es von Vorteil, in jede Ringscheibe ein am Außenrand angelenktes Auge zur Betätigung des Spreizrings im Sinne einer Schere aufweist. Insoweit könnte man die Spreizeinrichtung wie eine Schere bedienen, müßte lediglich zwei Finger in die Augen einführen und eine Schneidbewegung bzw. ein Zusammendrücken der Augen vornehmen. Dabei ist es von ganz besonderem Vorteil, wenn die Spreizeinrichtung in jeder Winkelstellung der Ringscheiben, d.h. in jede Position der Spreizfinger, arretierbar ist, um nämlich beide Hände zur Hygienebehandlung und anschließenden Katheterisierung verfügbar zu haben. Dazu könnte die Spreizeinrichtung als vorzugsweise federbeaufschlagtes, selbsthemmendes System ausgebildet sein, so daß ein Entspannen der Öffnung des Kondoms ausschließlich durch eine bewußt hervorgerufene entgegengesetzten Bewegung der Ringscheiben erfolgen kann.

Wie bereits zuvor erwähnt, handelt es sich bei der Spreizeinrichtung um ein mehrfach bzw. dauerhaft verwendbares Werkzeug oder Hilfsmittel. Insoweit ist es von Vorteil, wenn die Spreizeinrichtung aus einem sterilisierbaren Material besteht, so beispielsweise aus Metall oder gar rostfreiem Stahl. Ebenso ist es jedoch möglich, die Spreizeinrichtung im wesentlichen aus Kunststoff zu fertigen, wobei sich spritzgußtechnisch bearbeitbarer Kunststoff aus fertigungstechnischen Gesichtspunkten ganz besonders eignet.

Ein weiterer wesentlicher Bestandteil der Vorrichtung zur kombinierten Urinableitung und Katheterisierung ist das Kondom mit der im vorderen Bereich angelegten Öffnung, die zur Vermeidung eines ungewollten Ausreißens in ganz besonders vorteilhafter Weise mit einem Verstärkungswulst ausgestattet ist. Alternativ oder zusätzlich könnte der Bereich des Kondoms um die Öffnung herum dicker ausgeführt sein, so daß auch insoweit ein ungewolltes Ausreißen bei der Handhabung mit der Spreizeinrichtung wirksam vermieden ist.

Der dritte wesentliche Bestandteil der erfindungsgemäßen Vorrichtung ist die Anschlußeinrichtung, die zur Urinableitung zwischen den Katheterisierphasen und zur Freigabe der Öffnung des Kondom zum Katheterisieren dient. Diese Anschlußeinrichtung umfaßt ein durch die Öffnung des Kondoms hindurchsteckbares, an der Innenwandung des Kondoms um die Öffnung herum zur Anlage kommendes Einsteckteil und ein von außen gegen das Einsteckteil drückbares Klemmteil, wodurch ein abdichtender Anschluß des urinableitenden System an das Kondom gewährleistet ist. Das Klemmteil könnte dabei federkraftbeaufschlagt gegen das Einsteckteil drücken.

Des weiteren ist es zum materiaischonenden Anschluß an das Kondom von ganz besonderem Vorteil, wenn das Einsteckteil einsteckseitig eine Einfühmase und eine sich konisch zum freien Ende hin erweiternde Anlagefläche zur Anlage an die Innenwandung des Kondoms aufweist. Entsprechend könnte das Klemmteil eine dazu in etwa komplementäre Klemmfläche oder einen Klemmring bzw. eine kreisringförmige Klemmwulst aufweisen, wobei im Falle zweier Flächen diese in etwa parallel zueinander oder mit geringer Neigung zueinander verlaufen sollten.

Das Einsteckteil könnte anschlußseitig einen Anschlußstutzen zum Aufstecken einer vorzugsweise als Schlauch ausgeführten, zu dem Urinsammelbehältnis führenden Leitung aufweisen. Entsprechend könnte das Klemmteil über den Anschlußstutzen auf das Einsteckteil aufgeschoben sein, wobei das Klemmteil gegen die Kraft eines elastischen Mittels, vorzugsweise gegen die Kraft einer Feder, aus der Klemmposition von der Anlagefläche weg bewegbar sein könnte.

Unter Zugrundelegung der voranstehend genannten Konstruktion könnte die Anschlußeinrichtung ein Betätigungsorgan zum Lösen des Klemmteils aufweisen, wobei das Betätigungsorgan der Federkraft entgegen wirkt.

Zu der voranstehend genannten Funktion könnte die Anschlußeinrichtung ein von außen betätigbares Verschlußorgan umfassen, so daß der Durchfluß durch die Anschlußeinrichtung absperrbar ist. Ein Verschließen der Anschlußeinrichtung könnte dann erwünscht sein, wenn der Schlauch vom Anschlußstutzen gezogen werden soll. Jedenfalls erhöht die Vorkehrung eines Verschlußorgans die Flexibilität des Systems ganz erheblich.

Auch die Anschlußeinrichtung könnte im wesentlichen aus Metall, vorzugsweise aus rostfreiem Edelstahl, gefertigt sein. Die hygienische Handhabung der Anschlußeinrichtung wäre dadurch auf jeden Fall begünstigt. Jedoch ist es ohne weiteres auch möglich, die Anschlußeinrichtung im wesentlichen aus Kunststoff herzustellen, und zwar vorzugsweise aus spritzgußtechnisch verarbeitbarem Kunststoff, wodurch eine kostengünstige Herstellung auf spritzgußtechnischem Wege möglich ist.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung eines Ausführungsbeispiels der Erfindung anhand der Zeichnung zu verweisen. In Verbindung mit der Erläuterung des bevorzugten Ausführungsbeispiels der Erfindung anhand der Zeichnung werden auch im allgemeinen bevorzugte Ausgestaltungen und Weiterbildungen der Lehre erläutert. In der Zeichnung zeigt:
- Fig. 1 bis 12: in swchematischen Darstellungen ein Ausführungsbeispiel einer erfindungsgemäßen Vorrichtung zur kombinierten Urinableitung und Katheterisierung inkontinenter männlicher Personen, und zwar in einzelnen Schritten im Verlaufe der Handhabung vom Trennen der Anschlußeinrichtung vom angelegten Kondom über die Handhabung der Spreizeinrichtung über das Katheterisieren bis hin zum Schließen der Öffnung des Kondoms durch erneutes Festlegen der Anschlußeinrichtung zur anschließenden Urinableitung,
- Fig. 13: in schematischer Draufsicht ein Ausführungsbeispiel einer Spreizeinrichtung,
- Fig. 14: in schematischer Seitenansicht, geschnitten, den Gegenstand aus Fig. 13,
- Fig. 15: in schematischen Draufsicht den Gegenstand aus Fig. 13 mit auseinandergefahrenen Spreizfingern,
- Fig. 16: in schematischer Seitenansicht, geschnitten, den Gegenstand aus Fig. 15,
- Fig. 17: in schematischen Seitenansicht ein Ausführungsbeispiel einer Anschfußeinrichtung,
- Fig. 18: in schematischer Seitenansicht, teilweise geschnitten, den Gegenstand aus Fig. 17 um 90° gedreht und
- Fig. 19: den Gegenstand aus Fig. 17 und 18 in einer Vorderansicht.

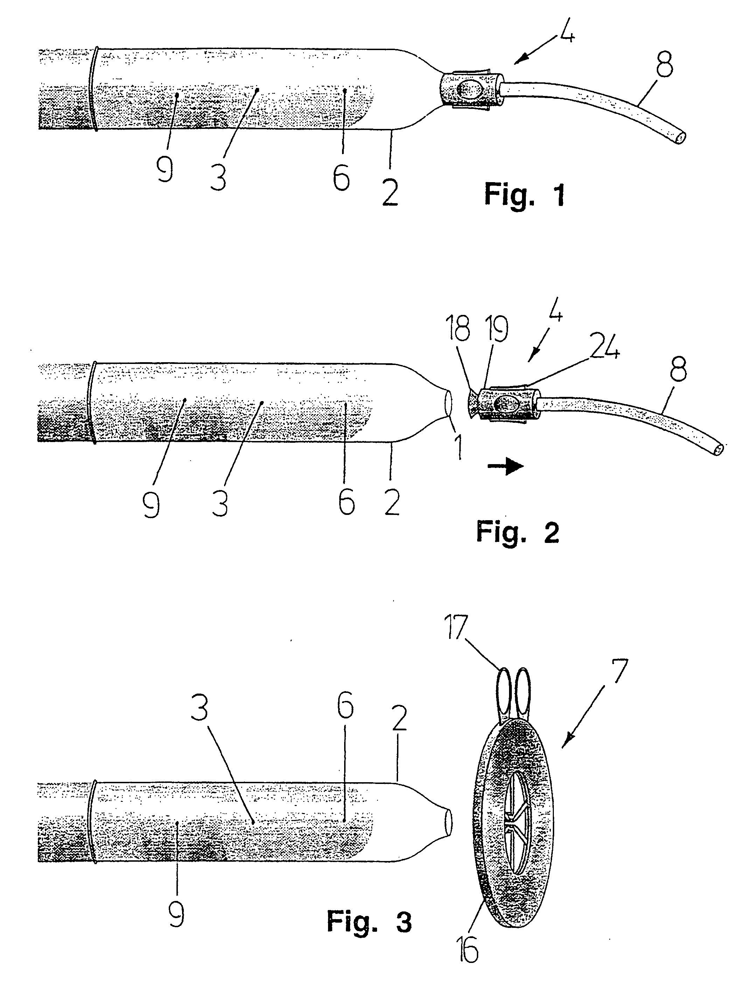

Die Figuren 1 bis 12 zeigen die Anwendung einer erfindungsgemäßen Vorrichtung zur kombinierten Urinableitung und Katheterisierung inkontinenter männlicher Personen, wobei es sich hier insbesondere um querschnittgefähmte männliche Personen handelt. Zu der Vorrichtung gehören ein im vorderen Bereich eine Öffnung 1 aufweisendes Kondom 2, das am Penisschaft 3 festgeklebt wird. An die Öffnung 1 des Kondoms 2 ist eine Anschlußeinrichtung 4 zum lösbaren Verbinden mit einem in den Figuren nicht gezeigten Urinsammelbehältnis vorgesehen. Ein Katheter 5 dient zum Selbstkatheterisieren durch den Patienten, wobei davor die Anschlußeinrichtung 4 zu entfernen und eine Reinigung der Eichel 6 bzw. eine Desinfizierung vorzunehmen ist.

Erfindungsgemäß ist eine zum Dehnen der Öffnung 1 des Kondoms 2 dienende Spreizeinrichtung 7 vorgesehen, mit deren Hilfe die Öffnung 1 soweit dehnbar ist, daß das Kondom 2 von der Öffnung 1 her gemeinsam mit der Spreizeinrichtung 7 über die Eichel 6 und zumindest über einen Teil des Penisschafts 3 hinweg dem Körper des Patienten entgegen schiebbar ist.

Fig. 1 zeigt den Zustand der an die Öffnung 1 des Kondoms 2 angeschlossene Anschlußeinrichtung 4, an die wiederum ein zu dem nicht gezeigten Urinsammelbehältnis führender Schlauch 8 angeschlossen ist. Das Kondom 2 ist in einem kreisringförmigen Klebebereich 9 mittels Klebstoff mit dem Penisschaft 3 rückflußsicher verbunden.

Gemäß der Darstellung in Fig. 2 ist die Anschlußeinrichtung 4 aus der Öffnung 1 des Kondoms 2 herausgelöst, so daß die Öffnung 1 frei liegt.

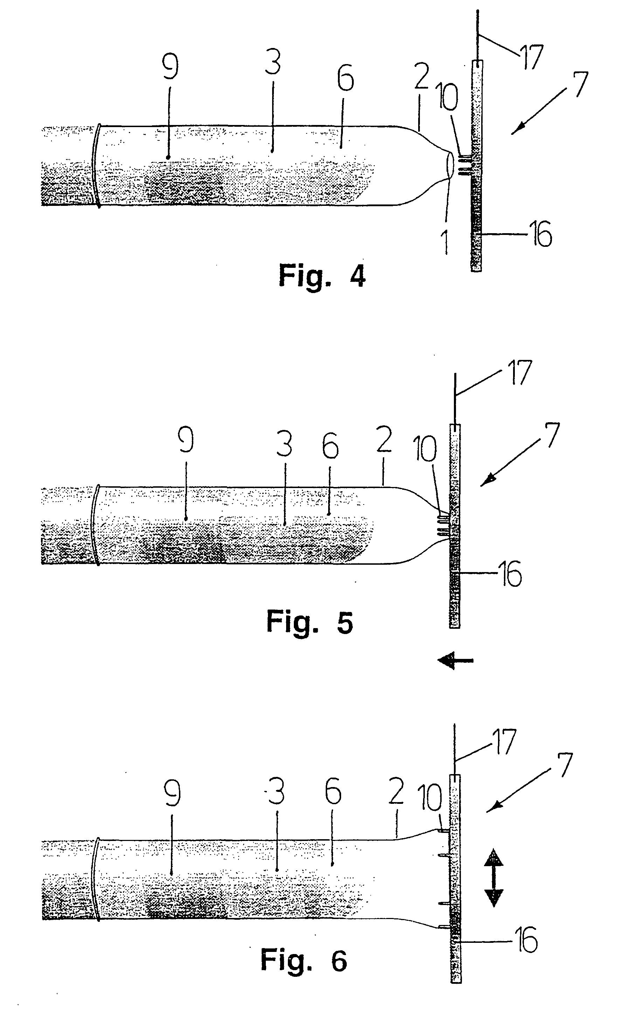

Die Fig. 3 und 4 zeigen den Zustand kurz vor dem Anlegen der Spreizeinrichtung 7, wobei dort die Öffnung 1 immer noch frei liegt.

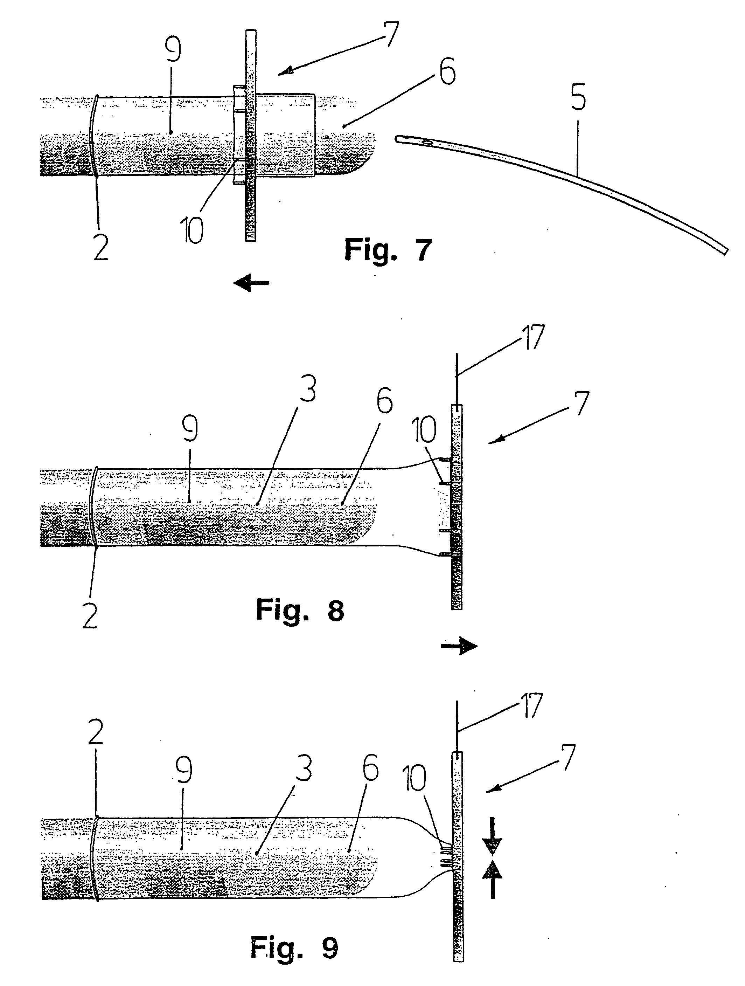

Gemäß der Darstellung in Fig. 5 ist die Spreizeinrichtung 7 mit mehreren Spreizfingem 10 in die Öffnung 1 eingeführt. Durch Betätigen der Spreizeinrichtung 7 werden die Spreizfinger 10 auseinander verlagert und dehnen die Öffnung 1 so weit, daß die gedehnt Öffnung 1 und somit der vordere Bereich des Kondoms 2 gemeinsam mit der Spreizeinrichtung 7 über die Eichel 6 hinweg entlang dem Penisschaft 3 zum Körper hin zurückgeschoben werden kann. Gemäß der Darstellung in Fig. 7 liegt nach dieser Handhabung die Eichel 6 frei und kann hygienisch behandelt bzw. desinfiziert werden. In Fig. 7 ist des weiteren angedeutet, daß nach Freilegen der Eichel 6 und nach der hygienischen Behandlung die Katheterisierung mittels des Katheters 5 möglich ist.

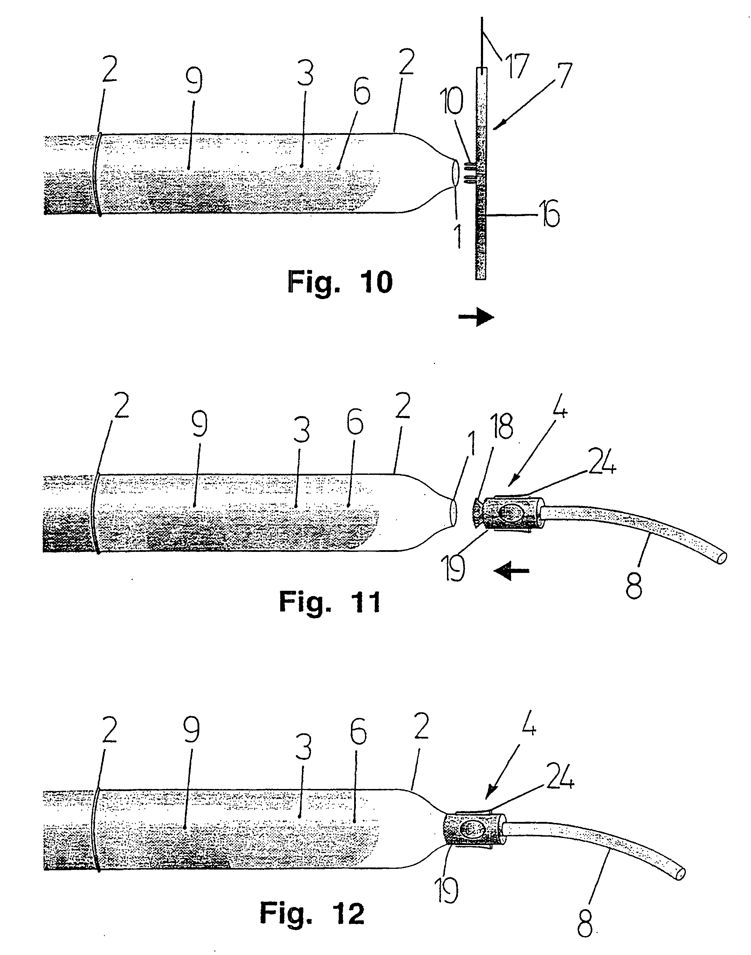

Fig. 8 zeigt die sich anschließende Situation, bei der die Spreizeinrichtung 7 wieder über die Eichel 6 hinweg nach vorn geschoben ist, wobei jedoch die Öffnung 1 mittels der Spreizfinger 10 immer noch gedehnt ist. Durch Betätigen der Spreizeinrichtung 7 verlagern sich die Spreizfinger 10 zur Mitte hin, wodurch eine Entlastung des Kondoms 2 im Bereich der Öffnung 1 stattfindet. Nach fölligem Zusammenfahren der Spreizfinger gemäß der Darstellung in Fig. 9 läßt sich die Spreizeinrichtung 7 gemäß der Darstellung in Fig. 10 und 11 entnehmen, so daß die Anschlußeinrichtung 4 mit dem urinableitenden Schlauch 8 wieder in die Öffnung 1 eingeführt und dort abdichtend festgelegt werden kann, wie dies in Fig. 12 letztendlich dargestellt ist.

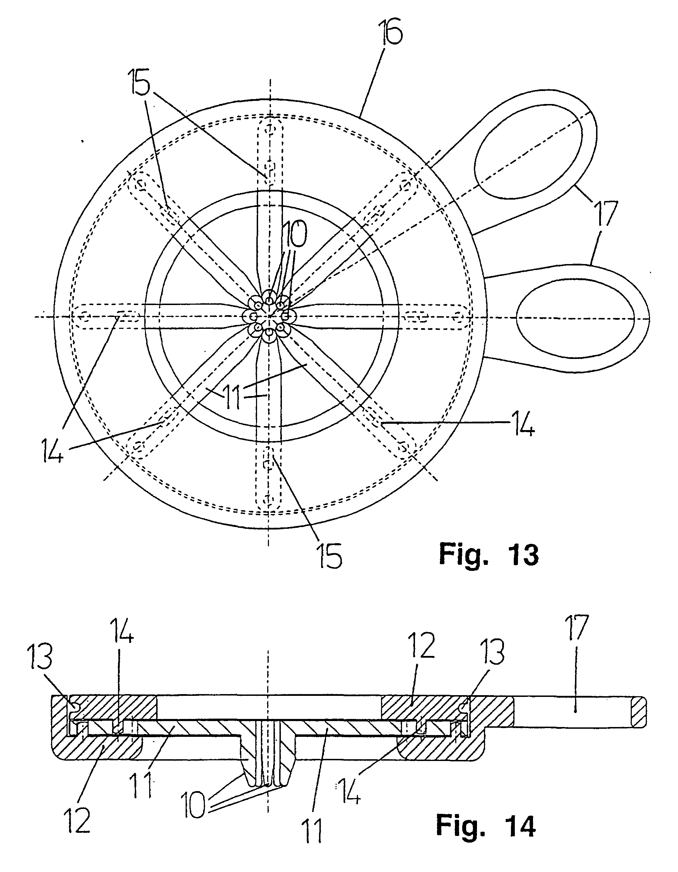

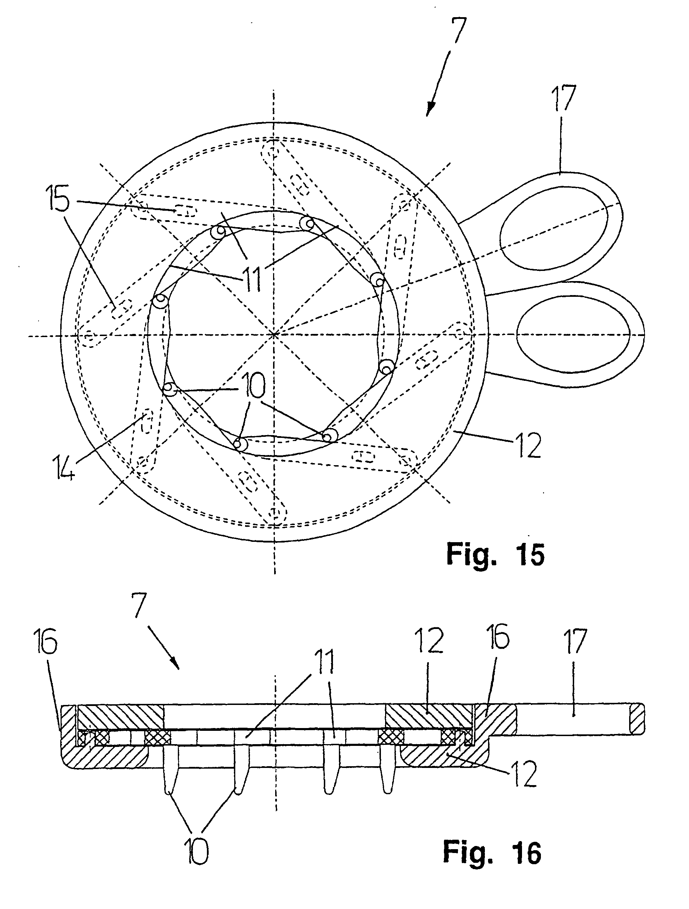

Die Fig. 13 bis 16 zeigen im Detail ein Ausführungsbeispiel einer Spreizeinrichtung 7, wobei dort die bereits zuvor erwähnten Spreizfinger 10 koaxial kreisringförmig angeordnet sind. Zum Dehnen der Öffnung 1 des Kondoms 2 lassen sich die Spreizfinger 10 auf einen größeren Radius auseinander bewegen, wie dies nämlich in Fig. 15 und 16 dargestellt ist. Die Fig. 13 bis 16 zeigen dabei gemeinsam, daß die Spreizfinger 10 blenden artig auseinander und aufeinander zu bewegbar sind.

Den Fig. 14 und 16 läßt sich entnehmen, daß die Spreizfinger 10 orthogonal von der Spreizeinrichtung 7 abragen und über eine Hebelanordnung an die Spreizeinrichtung 7 angelenkt sind. Im Konkreten ist jeder Spreizfinger 10 fest mit einem schwenkbaren Hebelarm 11 verbunden, wobei die Hebelanordnung und somit die Hebelarme 11 zwischen zwei Ringscheiben 12 geführt sind und wobei die Ringscheiben 12 gegenseitig in Führungsorgane 13 eingreifen und dabei drehbar miteinander verbunden sind.

Die Fig. 13 bis 16 zeigen besonders deutlich, daß die Hebelarme 11 an einer der Ringscheibe 12 schwenkbar angelenkt und an der anderen Ringscheibe 12 durch Führungsgelemente geführt sind, wobei es sich bei den Führungselementen um Stifte 14 handelt, die in Führungsschlitze 15 der Hebelarme 11 eingreifen.

Durch gegenseitiges Verdrehen der Ringscheiben 12 werden die Hebelarme 11 geschwenkt, wodurch wiederum die Spreizfinger 10 von einer kreisringförmigen Anordnung mit kleinem Durchmesser gemäß den Fig. 13 und 14 in eine kreisringförmige Anordnung mit größerem Durchmesser gemäß den Fig. 15 und 16 und umgekehrt verlagerbar sind.

In den Figuren 13 bis 16 ist des weiteren angedeutet, daß jede Ringscheibe 12 ein am Außenrand 16 angelenktes Auge 17 zur Betätigung der Spreizeinrichtung 7 im Sinne einer Schere aufweist. Bei beabstandeten Augen 17 gemäß der Darstellung in den Fig. 13 und 14 sind die Spreizfinger 10 in der inneren Position. Bei aneinanderliegenden Augen 17 gemäß der Darstellung in den Fig. 15 und 16 sind die Spreizfinger 10 auseinander verlagert, nämlich zum Dehnen der Öffnung 1.

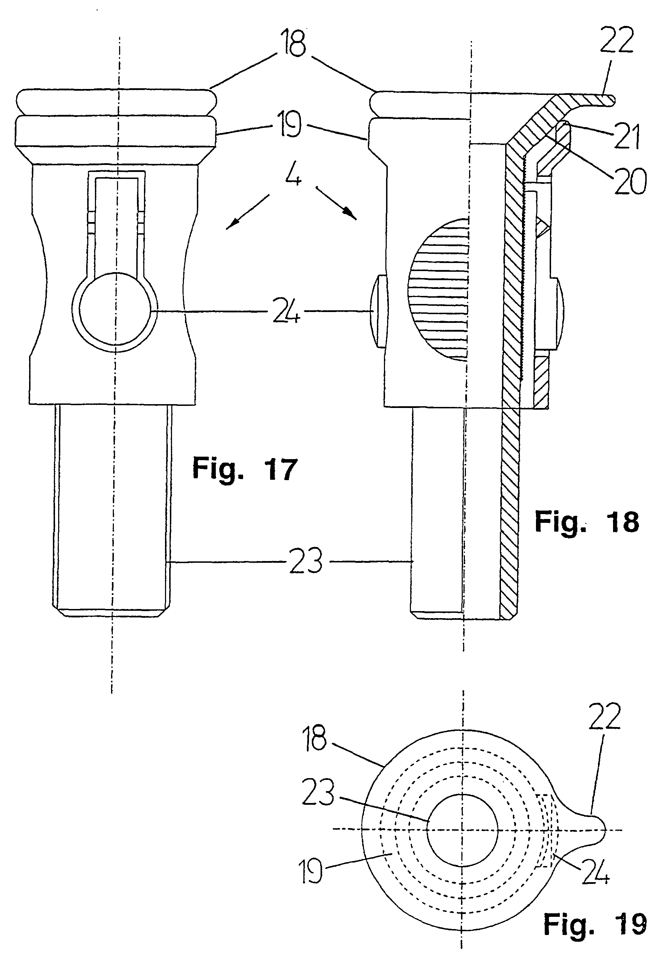

Die Fig. 17, 18 und 19 zeigen einen weiteren wesentlichen Bestandteil der erfindungsgemäßen Vorrichtung im Detail, nämlich die zum Anschluß an das nicht gezeigte Urinsammelbehältnis dienende Anschlußeinrichtung 4. Diese Anschlußeinrichtung 4 umfaßt ein durch die Öffnung 1 hindurchsteckbares, an der Innenwandung des Kondoms 2 um die Öffnung 1 herum zur Anlage kommendes Einsteckteil 18 und ein von außen gegen das Einsteckteil 18 drückbares Klemmteil 19. Das Klemmteil 19 kann federkraftbeaufschlagt gegen das Einsteckteil 18 drücken, wobei eine solche Vorkehrung den Fig. 17 bis 19 der Einfachheit halber nicht entnehmbar ist.

Die Fig. 17 und 18 zeigen gemeinsam, daß das Einsteckteil 18 einsteckseitig eine sich konisch zum freien Ende hin erweiternde Anlagefläche zur Anlage an die Innenwandung des Kondoms 2 und das Klemmteil 19 einen Klemmrand 21 aufweist, wobei es sich hier ebenso um eine in etwa komplementär ausgebildete Klemmfläche handeln kann. Der Klemmrand 21 dient bei dem hier gewählten Ausführungsbeispiel zum Festklemmen des Randbereichs um die Öffnung 1 herum.

Die Fig. 18 und 19 zeigen des weiteren, daß das Einsteckteil 18 eine Einfühmase 22 aufweist, die das Einführen bzw. Einschieben des Einsteckteils 18 in die Öffnung 1 aufgrund der nasenartigen Ausgestaltung erleichtert.

Die Fig. 17 und 18 zeigen des weiteren gemeinsam, daß das Einsteckteil 18 anschlußseitig einen Anschlußstutzen 23 zum Aufstecken des Schlauches 8 aufweist, der zu dem hier nicht gezeigten Urinsammelbehältnis führt.

Die Fig. 17 bis 19 lassen schließlich erkennen, daß das Klemmteil 19 auf das Einsteckteil 18 aufgeschoben ist. Gegen die Kraft eines nicht gezeigten elastischen Mittels läßt sich das Klemmteil 19 aus der Klemmposition von der Anlagefläche 20 weg bewegen, wobei die Anschlußeinrichtung 4 dazu ein Betätigungsorgan 24 zum Lösen bzw. Betätigen des Klemmteils 19 aufweist. Hinsichtlich weiterer Einzelheiten wird hier zur Vermeidung von Wiederholungen auf den allgemeinen Teil der Beschreibung verwiesen.

Abschließend sei ganz besonders darauf hingewiesen, daß das zuvor erörterte Ausführungsbeispiel lediglich zur Erörterung der beanspruchten Lehre dient, diese jedoch nicht auf das Ausführungsbeispiel einschränkt.

## Patentansprüche

1. Vorrichtung zur kombinierten Urinableitung und Katheterisierung inkontinenter männlicher Personen, insbesondere bei Querschnittslähmung, mit einem im vorderen Bereich eine Öffnung (1 ) aufweisenden Kondom (2) zum Festkleben am Penisschaft (3), einer abdichtend an die Öffnung (1) des Kondoms (2) anschliessbaren Anschlusseinrichtung (4) zum lösbaren Verbinden mit einem Urinsammelbehältnis und einem Katheter (5), wobei zum Katheterisieren die Anschlusseinrichtung (4) zu entfernen ist,
**gekennzeichnet durch** eine zum Dehnen der Öffnung (1 ) des Kondoms (2) dienende, als Spreizring ausgebildete Spreizeinrichtung (7), mit deren Hilfe die Öffnung (1) so weit dehnbar ist, dass das Kondom (2) von der Öffnung (1) her gemeinsam mit der Spreizeinrichtung (7) über die Eichel (6) und zumindest einen Teil des Penisschafts (3) hinweg dem Körper entgegen schiebbar ist, wobei die Spreizeinrichtung (7) in etwa koaxial kreisringförmig angeordnete Spreizfinger (10) zum Einführen in die ungedehnte Öffnung (1) des Kondoms (2) aufweist und wobei die Spreizfinger (10) zum Dehnen der Öffnung (1) auf einen grösseren Radius auseinander bewegbar sind.

2. Vorrichtung nach Anspruch 1, **dadurch gekennzeichnet, dass** die Spreizfinger (10) blendenartig auseinander und aufeinander zu bewegbar sind und vorzugsweise in etwa orthogonal von der Spreizeinrichtung (7) abragen und über eine Hebelanordnung an die Spreizeinrichtung (7) angelenkt sind.

3. Vorrichtung nach Anspruch 1 oder 2, **dadurch gekennzeichnet, dass** die Spreizeinrichtung (7) zwei gegeneinander drehbare Ringscheiben (12) umfasst, die gegebenenfalls gegenseitig in Führungsorgane (13) eingreifen und drehbar miteinander verbunden sind, wobei die Drehverbindung über Rastelemente, Rastnasen, Sprengringe oder dergleichen hergestellt sein können.

4. Vorrichtung nach Anspruch 2 oder 3, **dadurch gekennzeichnet, dass** die Hebelanordnung und somit die Hebelarme (11) zwischen den Ringscheiben (12) geführt sind, wobei die Hebelarme (11) vorzugsweise an einer der Ringscheiben (12) schwenkbar angelenkt und an der anderen Ringscheibe (12) durch Führungselemente geführt sind, so dass durch gegenseitiges Verdrehen der Ringscheiben (12) die Hebelarme (11) geschwenkt werden und dabei die Spreizfinger (10) von einer kreisringförmigen Anordnung mit kleinem Durchmesser in eine kreisringförmige Anordnung mit grösserem Durchmesser und umgekehrt verlagerbar sind und wobei die Führungselemente als die Hebelarme (11 ) führende Laschen oder als in Führungsschlitze (15) der Hebelarme (11) eingreifende Stifte (14) ausgeführt sind.

5. Vorrichtung nach Anspruch 3 oder 4, **dadurch gekennzeichnet, dass** die Ringscheiben (12) über einen um diese herum angeordneten Dreh-/Druckmechanismus auf Druck gegeneinander verdrehbar sind oder dass jede Ringscheibe (12) ein am Aussenrand (16) angelenktes Auge (17) zur Betätigung der Spreizeinrichtung (7) im Sinne einer Schere aufweist, wobei die Spreizeinrichtung (7) in jeder Winkelstellung der Ringscheiben (12), d.h. in jeder Position der Spreizfinger (10), arretierbar und gegebenenfalls als vorzugsweise federbeaufschlagtes, selbsthemmendes System ausgebildet sein kann.

6. Vorrichtung nach einem der Ansprüche 1 bis 5, **dadurch gekennzeichnet, dass** die Spreizeinrichtung (7) im wesentlichen aus Metall, vorzugsweise aus rostfreiem Stahl oder im wesentlichen aus Kunststoff, vorzugsweise spritzgusstechnisch, hergestellt ist.

7. Vorrichtung nach einem der Ansprüche 1 bis 6, **dadurch gekennzeichnet, dass** die Öffnung (1) des Kondoms (2) mit einem Verstärkungswulst ausgestattet ist, wobei der Bereich des Kondoms (2) um die Öffnung (1 ) herum dicker ausgeführt sein kann.

8. Vorrichtung nach einem der Ansprüche 1 bis 7, **dadurch gekennzeichnet, dass** die Anschlusseinrichtung (4) ein durch die Öffnung (1) hindurchsteckbares, an der Innenwandung des Kondoms (2) um die Öffnung (1) herum zur Anlage kommendes Einsteckteil (18) und ein von aussen gegen das Einsteckteil (18) drückbares Klemmteil (19) umfasst, welches vorzugsweise federkraftbeaufschlagt gegen das Einsteckteil (18) drückt, wobei das Einsteckteil (18) einsteckseitig eine sich konisch zum freien Ende hin erweiternde Anlagefläche (20) zur Anlage an die Innenwandung des Kondoms (2) und das Klemmteil (19) eine dazu in etwa komplementäre Klemmfläche oder einen Klemmrand (21) aufweisen kann.

9. Vorrichtung nach Anspruch 8, **dadurch gekennzeichnet, dass** das Einsteckteil (18) anschlussseitig einen Anschlussstutzen (23) zum Aufstecken einer vorzugsweise als Schlauch (8) ausgeführten, zu dem Urinsammelbehältnis führenden Leitung aufweist, wobei das Klemmteil (19) auf das Einsteckteil (18) aufgeschoben sein kann.

10. Vorrichtung nach Anspruch 8 oder 9, **dadurch gekennzeichnet, dass** das Klemmteil (19) gegen die Kraft eines elastischen Mittels, vorzugsweise gegen die Kraft einer Feder, aus der Klemmposition von der Anlagefläche weg bewegbar ist und wobei die Anschlusseinrichtung (4) ein Betätigungsorgan (24) zum Lösen des Klemmteils (19) aufweisen kann, welches der Federkraft entgegenwirkt.

11. Vorrichtung nach einem der Ansprüche 8 bis 10, **dadurch gekennzeichnet, dass** die Anschlusseinrichtung (4) ein von aussen betätigbares Verschlussorgan umfasst.

12. Vorrichtung nach einem der Ansprüche 8 bis 11, **dadurch gekennzeichnet, dass** die Anschlusseinrichtung (4) im wesentlichen aus Metall, vorzugsweise aus rostfreiem Edelstahl oder im wesentlichen aus Kunststoff, vorzugsweise spritzgusstechnisch, gefertigt ist.

## Claims

1. Device for the combined drainage of urine and catheterisation of incontinent male persons, in particular in the case of paraplegia, having a condom (2) which has an opening (1) in the front region for adhesive bonding to the penis body (3), a connection device (4), which can be connected in a sealing manner to the opening (1) of the condom (2), for releasable connection to a urine collection container, and having a catheter (5), the connection device (4) having to be removed for catheterisation,
**characterised by** an expansion device (7) which is used to expand the opening (1) of the condom (2) and which is in the form of an expansion ring and by means of which the opening (1) can be expanded to such an extent that the condom (2) can be pushed, starting from the opening (1) together with the expansion device (7), over the glans (6) and at least a portion of the penis body (3) towards the body, the expansion device (7) having expansion fingers (10), which are arranged substantially in a coaxial and annular manner, for insertion into the non-expanded opening (1) of the condom (2), and the expansion fingers (10) being able to be moved apart from each other in order to expand the opening (1) to a larger radius.

2. Device according to claim 1, **characterised in that** the expansion fingers (10) can be moved apart from and towards each other in the manner of a diaphragm and preferably project substantially orthoganally from the expansion device (7) and are articulated to the expansion device (7) by means of a lever arrangement.

3. Device according to claim 1 or 2, **characterised in that** the expansion device (7) comprises two annular discs (12) which can be rotated relative to each other and which optionally engage mutually in guide members (13) and which are connected to each other rotatably, the rotational connection being able to be produced by means of locking elements, locking projections, snap rings, or the like.

4. Device according to claim 2 or 3, **characterised in that** the lever arrangement, and consequently the lever arms (11), are guided between the annular discs (12), the lever arms (11) preferably being articulated pivotably to one of the annular discs (12) and being guided by guide elements on the other annular disc (12) so that the lever arms (11) are pivoted by mutual rotation of the annular discs (12) and the expansion fingers (10) can be displaced from an annular arrangement having a small diameter into an annular arrangement having a larger diameter, and vice versa, and the guide elements being constructed in the form of tabs which guide the lever arms (11) or in the form of pins (14) which engage in guide slots (15) of the lever arms (11).

5. Device according to claim 3 or 4, **characterised in that** the annular discs (12) can be rotated relative to each other in response to pressure by means of a rotation/pressure mechanism, which is arranged around the discs (12), or **in that** each annular disc (12) has an eye (17), which is articulated to the outer edge (16), for activating the expansion device (7) in the manner of a pair of scissors, the expansion device (7) being able to be lockable in any angular position of the annular discs (12), that is to say, in each position of the expansion fingers (10), and optionally being able to be in the form of a preferably resiliently biased, self-locking system.

6. Device according to any one of claims 1 to 5, **characterised in that** the expansion device (7) is produced substantially from metal, preferably from stainless steel, or substantially from plastics material, preferably using the injection-moulding technique.

7. Device according to any one of claims 1 to 6, **characterised in that** the opening (1) of the condom (2) is provided with a reinforcement bead, the region of the condom (2) around the opening (1) being able to be constructed so as to be thicker.

8. Device according to any one of claims 1 to 7, **characterised in that** the connection device (4) comprises an insert member (18), which can be fitted through the opening (1) and which abuts the inner wall of the condom (2) around the opening (1), and a clamping member (19), which can be pressed from outside against the insert member (18) and which preferably presses against the insert member (18) in a resiliently biased manner, it being possible for the insert member (18) to have, at the insertion side, a contact face (20), which widens conically towards the free end, for abutment against the inner wall of the condom (2) and for the clamping member (19) to have a clamping face which is substantially complementary thereto or a clamping edge (21).

9. Device according to claim 8, **characterised in that** the insert member (18) has, at the connection side, a connection piece (23) for the attachment of a line, which is preferably in the form of a hose (8) and which leads to the urine collection container, the clamping member (19) being able to be fitted onto the insert member (18).

10. Device according to claim 8 or 9, **characterised in that** the clamping member (19) can be moved away from the contact face from the clamping position counter to the force of a resilient means, preferably counter to the force of a spring, and the connection device (4) being able to have an actuation element (24) for releasing the clamping member (19), which element (24) counter-acts the resilient force.

11. Device according to any one of claims 8 to 10, **characterised in that** the connection device (4) comprises an externally actuatable closure element.

12. Device according to any one of claims 8 to 11, **characterised in that** the connection device (4) is produced substantially from metal, preferably from stainless high-quality steel, or substantially from plastics material, preferably using the injection-moulding technique.

## Revendications

1. Dispositif pour la dérivation urinaire et le cathétérisme combinés de sujets mâles incontinents, en particulier dans le cas d'une paralysie transversale, avec un préservatif (2) présentant une ouverture (1 ) dans la zone antérieure pour le collage de fixation à la verge du pénis (3), un dispositif de raccordement (4) pouvant être raccordé de manière étanche à l'ouverture (1) du préservatif (2) pour une liaison amovible avec un récipient collecteur d'urine et un cathéter (5), le dispositif de raccordement (4) devant être enlevé pour le cathétérisme,
**caractérisé par** un dispositif d'écartement (7), réalisé sous forme d'anneau d'écartement, servant à étirer l'ouverture (1 ) du préservatif (2), à l'aide duquel l'ouverture (1) peut être étirée suffisamment loin pour que le préservatif (2) puisse être glissé depuis l'ouverture (1), en même temps que le dispositif d'écartement (7), sur le gland (6) et au moins par-dessus une partie de la verge du pénis (3) vers le corps, le dispositif d'écartement (7) présentant des doigts d'écartement (10) disposés sensiblement coaxialement en forme de cercle pour l'introduction dans l'ouverture (1) non étirée du préservatif (2) et les doigts d'écartement (10) pouvant être écartés les uns des autres à un diamètre supérieur pour étirer l'ouverture (1).

2. Dispositif selon la revendication 1,
**caractérisé en ce que** les doigts d'écartement (10) sont mobiles à écartement les uns des autres et les uns vers les autres à la manière d'un diaphragme et font saillie de préférence sensiblement orthogonalement depuis le dispositif d'écartement (7) et sont articulés sur le dispositif d'écartement (7) par l'intermédiaire d'un arrangement de leviers.

3. Dispositif selon la revendication 1 ou 2,
**caractérisé en ce que** le dispositif d'écartement (7) comprend deux disques annulaires (12) pivotant l'un par rapport à l'autre, qui sont en prise le cas échéant réciproquement dans des organes de guidage (13) et sont reliés l'un à l'autre en rotation, la liaison en rotation pouvant être formée par des éléments d'encliquetage, des nez d'encliquetage, des joncs et similaires.

4. Dispositif selon la revendication 2 ou 3,
**caractérisé en ce que** l'arrangement de leviers et ainsi les bras des leviers (11 ) sont guidés entre les disques annulaires (12), les bras des leviers (11) étant de préférence articulés à pivotement sur l'un des disques annulaires (12) et guidés sur l'autre disque annulaire (12) par des éléments de guidage de telle manière que les bras des leviers (11 ) sont déplacés en pivotement par une rotation réciproque des disques annulaires (12) et que les doigts d'écartement (10) peuvent ainsi être déplacés d'une disposition en forme circulaire à faible diamètre à une disposition en forme circulaire à plus grand diamètre et inversement et les éléments de guidage étant réalisés sous forme de colliers guidant les bras des leviers (11 ) ou sous forme de broches (14) en prise dans des fentes de guidage (15) des bras des leviers (11 ).

5. Dispositif selon la revendication 3 ou 4,
**caractérisé en ce que** les disques annulaires (12) peuvent être entraînés en pivotement réciproquement par pression par l'intermédiaire d'un mécanisme de rotation/pression disposé autour de ceux-ci ou que chaque disque annulaire (12) présente un oeillet (17) articulé au bord extérieur (16) pour actionner le dispositif d'écartement (7) à la manière de ciseaux, le dispositif d'écartement (7) pouvant, dans chaque position angulaire des disques annulaires (12), c'est-à-dire dans chaque position des doigts d'écartement (10), être arrêté et pouvant être réalisé le cas échéant sous forme de système auto-bloquant, de préférence rappelé par ressort.

6. Dispositif selon l'une des revendications 1 à 5,
**caractérisé en ce que** le dispositif d'écartement (7) est fabriqué essentiellement en métal, de préférence en acier inoxydable, ou essentiellement en matière synthétique, de préférence par technique de moulage par injection.

7. Dispositif selon l'une des revendications 1 à 6,
**caractérisé en ce que** l'ouverture (1) du préservatif (2) est équipée d'un bourrelet de renforcement, la zone du préservatif (2) tout autour de l'ouverture (1) pouvant être réalisée plus épaisse.

8. Dispositif selon l'une des revendications 1 à 7,
**caractérisé en ce que** le dispositif de raccordement (4) comprend une pièce d'enfichage (18) pouvant être enfoncée à travers l'ouverture (1) et venant en appui sur la paroi intérieure du préservatif (2) tout autour de l'ouverture (1), ainsi qu'une pièce de serrage (19) pouvant être pressée depuis l'extérieur contre la pièce d'enfichage (18) et qui presse, de préférence par action d'une force de ressort, contre la pièce d'enfichage (18), la pièce d'enfichage (18) pouvant présenter du côté d'enfichage une surface d'appui (20) s'élargissant coniquement en direction de l'extrémité libre pour l'appui sur la paroi intérieure du préservatif (2) et la pièce de serrage (19) pouvant présenter une surface de serrage sensiblement complémentaire à celle-ci ou un bord de serrage (21).

9. Dispositif selon la revendication 8,
**caractérisé en ce que** la pièce d'enfichage (18) présente du côté de raccordement un manchon de raccordement (23) pour monter une conduite conduisant au récipient collecteur d'urine, de préférence réalisée sous forme de tuyau (8), la pièce de serrage (19) pouvant être déplacée en coulissement sur la pièce d'enfichage (18).

10. Dispositif selon la revendication 8 ou 9,
**caractérisé en ce que** la pièce de serrage (19) peut être déplacée hors de la position de serrage en s'écartant de la surface d'appui, contre la force d'un moyen élastique, de préférence contre la force d'un ressort, et le dispositif de raccordement (4) pouvant présenter un organe d'actionnement (24) pour libérer la pièce de serrage (19) et qui agit à l'encontre de la force de ressort.

11. Dispositif selon l'une des revendications 8 à 10,
**caractérisé en ce que** le dispositif de raccordement (4) comprend un organe de fermeture pouvant être actionné de l'extérieur.

12. Dispositif selon l'une des revendications 8 à 11,
**caractérisé en ce que** le dispositif de raccordement (4) est fabriqué essentiellement en métal, de préférence en acier spécial inoxydable, ou essentiellement en matière synthétique, de préférence par technique de moulage par injection.
